# EUROPÄISCHE PATENTSCHRIFT

(11) **EP 3 152 187 B1**
(45) Veröffentlichungstag und Bekanntmachung des Hinweises auf die Patenterteilung: **03.01.2018**
(21) Anmeldenummer: 15724991.3
(22) Anmeldetag: 21.05.2015
(51) Int. Cl.: C07C 67/10, C07C 67/60, C07C 51/56, C07C 51/573, C07C 69/24, C07C 69/01, C07C 53/126, B01D 3/00, C07C 67/54

(54) **VERFAHREN ZUR TRENNUNG VON HOCHSIEDENDEN CARBONSÄUREVINYLESTER/CARBONSÄURE-GEMISCHEN**
METHOD FOR SEPARATING HIGH-BOILING CARBOXYLIC ACID VINYL ESTER/CARBOXYLIC ACID MIXTURES
PROCÉDÉ POUR LA SÉPARATION DE MÉLANGES ESTER VINYLIQUE D'ACIDE CARBOXYLIQUE/ACIDE CARBOXYLIQUE À POINT D'ÉBULLITION ÉLEVÉ

(30) Priorität: 06.06.2014 DE 102014210835
(43) Veröffentlichungstag der Anmeldung: 12.04.2017
(73) Patentinhaber: Wacker Chemie AG, 81737 München (DE)
(72) Erfinder: GIGLER, Peter, 85221 Dachau (DE); STOHRER, Jürgen, 82049 Pullach (DE)
(74) Vertreter: Schuderer, Michael
(86) Internationale Anmeldenummer: PCT/EP2015/061231
(87) Internationale Veröffentlichungsnummer: WO 2015/185365

(56) Entgegenhaltungen:
- WO-A1-2011/139360
- GB-A- 869 830
- JP-A- H05 279 296
- US-A- 3 188 319

## Beschreibung

Die Erfindung betrifft ein Verfahren zur Trennung von hochsiedenden Carbonsäurevinylester/Carbonsäure-Gemischen.

Die destillative Trennung von Gemischen aus hochsiedenden Carbonsäurevinylestern und hochsiedenden Carbonsäuren wird mit wachsendem Molekulargewicht der Komponenten zunehmend erschwert. Unter hochsiedenden Carbonsäurevinylestern und hochsiedenden Carbonsäuren sind Carbonsäurevinylester und Carbonsäuren zu verstehen, welche jeweils mindestens 12 C-Atome im Carbonsäurerest aufweisen. Der Grund für deren schwierige Auftrennung sind sehr hohe und nahe beieinander liegende Siedepunkte. Deutlich wird dies zum Beispiel an den Siedepunkten von Palmitinsäurevinylester (346°C bei 1 bar abs.) und Palmitinsäure (351 °C bei 1 bar abs.) bzw. Stearinsäurevinylester (385°C bei 1 bar abs.) und Stearinsäure (361 °C bei 1 bar abs.). Eine destillative Auftrennung solcher Komponenten mit sehr hohen und nahe beieinander liegenden Siedepunkten, insbesondere wenn Carbonsäuren bzw. Carbonsäurevinylester unterschiedlicher Kettenlänge im Gemisch vorliegen, ist im großtechnischen Maßstab nicht möglich.

Solche Gemische aus Carbonsäurevinylestern und Carbonsäuren fallen zum Beispiel bei der Umvinylierung von Carbonsäuren an, wenn die Eduktcarbonsäure mindestens 12 C-Atome aufweist. Unter Umvinylierung versteht man die Übertragung einer Vinyl-Einheit eines Eduktvinylesters (1V) auf eine Eduktcarbonsäure (2S) unter Generierung eines Produktvinylesters (2V) und der korrespondierenden Säure des Eduktvinylesters (1S).

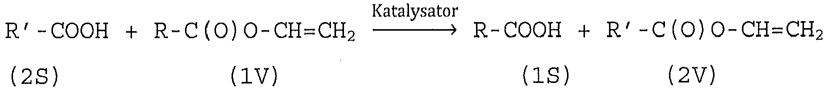

Nach der destillativen Abtrennung leichtsiedender Komponenten (meist der Eduktvinylester, zum Beispiel Vinylacetat, und dessen korrespondierende Säure, zum Beispiel Essigsäure) erhält man das zu trennende Gemisch aus Carbonsäurevinylester und Carbonsäure.

Die Herstellung hochsiedender Carbonsäurevinylester mittels Umvinylierung wird in den Beispielen zahlreicher Patentpublikationen beschrieben. Pd-katalysierte Umvinylierungen zur Darstellung von Vinylpalmitat und Vinylstearat werden in der WO 2011/139360 A1 (Beispiele 69 und 70), der JP 05-279296 A (Beispiel 1) und der US 5,214,172 A (Beispiele 19-40 und 56-57) beschrieben, während die Hg-katalysierte Variante in der US 3,158,633 A (Beispiele 1 - 5), der GB 827718 A (Beispiel 15), Anwendung findet. In der WO 2011/139360 A1 wird auf die Aufarbeitung des Produktgemisches nicht näher eingegangen. Auch in der JP 05-279296 finden sich dazu keine Angaben. Die US 5,214,172 A befasst sich mit der Aktivität von Pd-Katalysatoren bei der Umvinylierung, ohne die Aufarbeitung des Produktgemisches zu behandeln. Auch in der US 3,158,633 A wird auf die Aufarbeitung des Reaktionsproduktes nicht eingegangen. In der GB 827718 wird erwähnt, dass der Produktvinylester abdestilliert werden kann, ohne konkrete Maßnahmen zu beschreiben.

Die Abtrennung von Stearinsäurevinylester über fraktionierte Destillation wird in der JP 07-138203 A (Beispiel 8), der US 4,425,277 A (Beispiel 24) und der US 3,188,319 A (Beispiel 2) genannt. Eine genaue Ausgestaltung dieses Trennverfahrens wird, ebenso wie eine chemische Analyse der Destillationsfraktionen nicht beschrieben. Lediglich in Beispiel 2 der US 3,188,319 A wird ein Siedepunkt des Produktes mit 203°C bei 5 mmHg (∼376°C bei 1 bar) angegeben. Es liegt nahe, dass in diesen Fällen ein Gemisch aus Stearinsäure und Stearinsäurevinylester abgetrennt wurde, da Stearinsäure (361°C bei 1 bar) einen niedrigeren Siedepunkt als der entsprechende Stearinsäurevinylester (385°C bei 1 bar) aufweist.

Das Patent US 3,000,918 A beansprucht ein Verfahren zur Trennung von Carbonsäurevinylester/Carbonsäure-Gemischen, bei dem die Carbonsäure durch Zugabe von Natriumhydroxid in das entsprechende, schwerflüchtige Na-Salz überführt wird, welches durch Filtration vom Carbonsäurevinylester abgetrennt werden kann. Dieser kann in der Folge destillativ gereinigt werden. Das gleiche Verfahren macht sich die US 3,179,641 A zur Darstellung von Phenylstearinsäurevinylester zunutze. Wie in Beispiel 1 beschrieben, wird nach Abtrennung von Vinylacetat und Essigsäure die verbleibende Phenylstearinsäure durch Zugabe von Natriumhydroxid in das schwerflüchtige Na-Salz überführt und der Vinylester destillativ abgetrennt. Die Abtrennung des Na-Salzes erfordert einen verfahrenstechnisch aufwendigen Fest-Flüssig-Trennschritt, was einen Nachteil dieses Verfahrens darstellt. Das Na-Salz kann zwar nach Rücküberführung in die Carbonsäure wieder eingesetzt werden, jedoch ist auch hierfür ein zusätzlich chemischer Prozessschritt nötig. Beide Schriften beschreiben weiterhin, dass es ohne vorherige Abtrennung der Eduktcarbonsäure bei der Destillation zu Zersetzungsreaktionen und Polymerisation des Vinylesters kommt.

Aus der GB 869830 A ist ein Verfahren zur Herstellung von Vinylstearat bekannt, bei dem Vinylstearat durch Extraktion mit einem aliphatischen Kohlenwasserstoff von Stearinsäure abgetrennt wird. Wahlweise kann anschließend eine weitere Aufreinigung durch verschiedene Adsorbentien erfolgen. Die Autoren beschreiben, dass dieses Verfahren im Falle der Verwendung kommerzieller Stearinsäure nur bis zu einem Palmitinsäure-Anteil von 30 % anwendbar ist, da die Extraktion mit zunehmendem Anteil an Palmitinsäure schwieriger wird. Carbonsäurevinylester/Carbonsäure-Gemische auf Basis kommerzieller Stearinsäure 50, die einen Palmitinsäureanteil von bis zu 50 Gew.-% aufweist, lassen sich mit diesem Verfahren folglich nicht trennen. Dieses Extraktionsverfahren wird weiterhin in folgenden Schriften beschrieben: US 2,997,494 A (Beispiel 1), US 2,997,495 A (Beispiel 7), GB 869828 A (Beispiel 1) und US 3,201,357 A (Beispiel 12).

Hochsiedende Carbonsäurevinylester lassen sich destillativ nicht mehr von den entsprechenden Carbonsäuren trennen. Dies ist vor allem für die Produkte aus Umvinylierungsreaktionen relevant, die auf hochsiedenden Carbonsäuren basieren, wie beispielsweise den kommerziell verfügbaren Mischungen aus Palmitin- und Stearinsäure. Neben einer Extraktionsmethode für Stearinsäurevinylester, der aus Stearinsäure mit einer Reinheit > 70 Gew.-% hergestellt wurde, beschreibt der Stand der Technik die reaktive Überführung der Carbonsäure in entsprechende, schwerflüchtige Na-Carboxylate. Diese erfordern jedoch einen aufwendigen Fest-Flüssig-Trennschritt sowie einen zusätzlichen chemischen Prozessschritt um das Na-Carboxylat in die Carbonsäure rück zu überführen. Ein breit anwendbares Verfahren zur Trennung hochsiedender Carbonsäurevinylester/Carbonsäure-Gemische, das ohne aufwendige Prozessschritte zur Feststoffabtrennung sowie Rücküberführung zur Carbonsäure auskommt, ist bislang unbekannt.

Es bestand daher die Aufgabe, ein Verfahren zur Trennung von hochsiedenden Carbonsäurevinylester/Carbonsäure-Gemischen zu entwickeln, welches sich einerseits durch eine breite Anwendbarkeit auf hochsiedende Carbonsäurevinylester bzw. Carbonsäuren oder entsprechende Mischungen auszeichnet und keinen aufwendigen Fest-Flüssig-Trennschritt erfordert.

Gegenstand der Erfindung ist ein Verfahren zur Trennung eines Gemisches enthaltend mindestens einen Carbonsäurevinylester der allgemeinen Formel R'-C(O)O-CH=CH₂ und mindestens eine Carbonsäure der allgemeinen Formel R'-COOH, wobei R' jeweils ein aliphatischer Rest mit 12 bis 22 C-Atomen, oder ein cycloaliphatischer Rest mit 12 bis 22 C-Atomen oder ein aromatischer Rest mit 12 bis 22 C-Atomen sein kann und R' gleich oder verschieden sein können, dadurch gekennzeichnet, dass die Carbonsäure in deren Anhydrid R-C(O)-O-C(O)-R' überführt wird, und der Carbonsäurevinylester anschließend abgetrennt wird.

Mit dem Verfahren können Carbonsäurevinylester der allgemeinen Formel R'-C(O)O-CH=CH₂, wobei R' ein aliphatischer Rest mit 12 bis 22 C-Atomen oder ein cycloaliphatischer Rest mit 12 bis 22 C-Atomen oder ein aromatischer Rest mit 12 bis 22 C-Atomen sein kann, aus einem Gemisch abgetrennt werden. Es können auch Carbonsäurevinylester mit verschiedenen Resten R' im Gemisch vorliegen. Beispiele sind Vinylester der Laurinsäure, Myristinsäure, Palmitinsäure, Stearinsäure, Naphtalincarbonsäure oder Gemische derselben. Besonders bevorzugt werden Vinylester der Carbonsäuren mit 15 bis 22 C-Atomen, wie Palmitinsäure und Stearinsäure, sowie Gemische derselben eingesetzt.

Carbonsäuren, welche im Gemisch mit dem Carbonsäurevinylester vorliegen können, sind Carbonsäuren der allgemeinen Formel R'-COOH, wobei R' ein aliphatischer Rest mit 12 bis 22 C-Atomen oder ein cycloaliphatischer Rest mit 12 bis 22 C-Atomen oder ein aromatischer Rest mit 12 bis 22 C-Atomen sein kann. Es können auch Carbonsäuren mit verschiedenen Resten R' im Gemisch vorliegen. Beispiele hierfür sind Laurinsäure, Myristinsäure,

Palmitinsäure, Stearinsäure, Naphtalincarbonsäure oder Gemische derselben. Besonders bevorzugt werden Fettsäuren mit 15 bis 22 C-Atomen, wie Palmitinsäure und Stearinsäure, sowie Gemische derselben.

Das Gemisch aus Carbonsäure R'-COOH und Carbonsäurevinylester R'-C(O)O-CH=CH₂ kann weitere Komponenten, beispielsweise aus einer Umvinylierungsreaktion, wie Katalysatoren, Anhydride, Säuren und polymere Bestandteile enthalten.

Der molare Anteil x der Carbonsäure R'-COOH, bezogen auf das Gemisch aus Carbonsäurevinylester R'-C(O)O-CH=CH₂ und Carbonsäure R'-COOH, kann 0 Mol-% < x < 100 % betragen, bevorzugt ist ein Anteil von 0,01 Mol-% ≤ x ≤ 50 Mol-%, besonders bevorzugt ein Verhältnis von 0,1 Mol-% ≤ x ≤ 30 Mol-%.

In einer ersten bevorzugten Ausführungsform erfolgt die Überführung der Carbonsäure R'-COOH in das entsprechende Anhydrid R'-C(O)-O-C(O)-R' durch Reaktion mit einem Anhydrid der allgemeinen Formel R*-C(O)-O-C(O)-R*, wobei R* gleich oder verschieden ist und einen aliphatischen Rest mit 1 bis 10 C-Atomen, oder einen cycloaliphatischen Rest mit bis zu 10 C-Atomen oder einen aromatischen Rest mit bis zu 10 C-Atomen darstellt. Beispiele hierfür sind die Anhydride folgender Säuren: Essigsäure, Propionsäure, n-Buttersäure, iso-Buttersäure, n-Valeriansäure, 2-Methylbuttersäure, 3-Methylbuttersäure, Pivalinsäure, Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure. Bevorzugt wird das Anhydrid der Essigsäure (Essigsäureanhydrid) eingesetzt.

Eingesetzt werden 0,1 bis 5 molare Äquivalente des Anhydrids R*-C(O)-O-C(O)-R*, bezogen auf die Carbonsäure R'-COOH. Bevorzugt werden 0,25 bis 3 molare Äquivalente des Anhydrids eingesetzt, besonders bevorzugt 0,5 bis 2 molare Äquivalente, jeweils bezogen auf die Carbonsäure R'-COOH.

Die Umsetzung des Gemisches enthaltend einen Carbonsäurevinylester R'-C(O)O-CH=CH₂ und eine Carbonsäure R'-COOH mit dem Anhydrid R*-C(O)-O-C(O)-R* erfolgt im Allgemeinen bei Temperaturen von 0°C bis 150°C, bevorzugt bei 30°C bis 120°C, besonders bevorzugt bei 50°C bis 100°C. Die Reaktionszeit beträgt im Allgemeinen bis zu 5 Stunden, bevorzugt bis zu 2 Stunden besonders bevorzugt 5 bis 60 Minuten. Der Druck, bei welchem die Umsetzung erfolgt, beträgt im Allgemeinen 0,001 bis 10 bar abs., bevorzugt 0,1 bis 2 bar, besonders bevorzugt wird bei Normaldruck (1 bar abs.) gearbeitet.

In einer zweiten bevorzugten Ausführungsform kann die Überführung der Carbonsäure R'-COOH in deren Anhydrid R'-C(O)-O-C(O)-R' durch Übergangsmetall-katalysierte Reaktivdestillation mit einem Carbonsäurevinylester der allgemeinen Formel R-C(O)O-CH=CH₂ erfolgen, wobei R ein aliphatischen Rest mit 1 bis 10 C-Atomen oder ein cycloaliphatischer Rest mit bis zu 10 C-Atomen oder ein aromatischer Rest mit bis zu 10 C-Atomen sein kann. Bevorzugt ist die Verwendung niedermolekularer Carbonsäurevinylester, wobei R ein Alkylrest mit 1 bis 6 C-Atomen ist. Besonders bevorzugt ist die Verwendung von Vinylacetat.

Als Katalysator bevorzugt werden Ru-Verbindungen oder Pd-Verbindungen, welche typischerweise für Umvinylierungsreaktionen eingesetzt werden. Geeignete Katalysatoren sind dem Fachmann bekannt, beispielsweise aus der WO 2011/139360 A1, der WO 2011/139361 A1 und dem US-Patent 4,981,973 auf deren diesbezügliche Offenbarung Bezug genommen wird. Bevorzugt werden Ru-Verbindungen eingesetzt. Besonders bevorzugt ist die Verwendung von Ru-Acetat oder einer aktiven Ru-Katalysatorlösung, wie sie dem Fachmann aus den Offenlegungsschriften DE 102014206915 und DE 102014206916 bekannt sind.

Das molare Verhältnis von Carbonsäurevinylester R-C(O)O-CH=CH₂ zu Carbonsäure R'-COOH kann 1 : 1 bis 40 : 1 betragen. Bevorzugt ist ein Verhältnis von Carbonsäurevinylester zu Carbonsäure von 10 : 1 bis 30 : 1, besonders bevorzugt ist ein Verhältnis von 15 : 1 bis 25 : 1.

Die Reaktivdestillation wird im Allgemeinen bei einer Temperatur von 100°C bis 180°C, vorzugsweise bei einer Temperatur von 120°C bis 160°C durchgeführt. Der Druck, bei welchem die Reaktivdestillation erfolgt, beträgt im Allgemeinen 1 bar abs.. Die Reaktion wird bevorzugt in einer Schutzgasatmosphäre, beispielsweise Stickstoff, in an sich bekannter Weise, durchgeführt.

Die Verweilzeit im Reaktor beträgt im Allgemeinen 1 bis 10 Stunden, vorzugsweise 3 Stunde bis 8 Stunden.

Als Reaktor kann eine Blasensäule oder ein kontinuierlicher Rührkessel eingesetzt werden. Bevorzugt wird ein kontinuierlicher Rührkessel eingesetzt.

Die Reaktivdestillation zur Überführung der Carbonsäure R'-COOH in das entsprechende Anhydrid R'-C(O)-O-C(O)-R' kann separat oder in Kombination mit einer als Reaktivdestillation ausgeführten Umvinylierungreaktion durchgeführt werden. In diesem Falle wird die Reaktivdestillation nach erfolgter Umvinylierungsreaktion so lange weitergeführt und die Carbonsäure R'-COOH dabei in das entsprechende Anhydrid R'-C(O)-O-C(O)-R' überführt, bis der Gehalt an restlicher Carbonsäure R'-COOH im Reaktionsgemisch vorzugsweise maximal 5 Gew.-% beträgt.

In beiden bevorzugten Ausführungsformen erfolgt nach Überführung der Carbonsäure R'-COOH in deren Anhydrid R'-C(O)-O-C(O)-R' die Auftrennung des Reaktionsgemisches aus Carbonsäurevinylester R'-C(O)O-CH=CH₂ und dem Anhydrid R'-C(O)-O-C(O)-R'. Das Gemisch kann gegebenenfalls weitere Komponenten wie Katalysatoren, Anhydride, Säuren und polymere Bestandteile enthalten. Die Auftrennung des Gemisches erfolgt bevorzugt durch Destillation. Druck und Temperatur der Destillation sowie die Auslegung der Destillationskolonnen hängen von den im Produktgemisch vorliegenden Komponenten ab und können beispielsweise mittels Routineversuchen vom Fachmann ermittelt werden.

Das erfindungsgemäße Verfahren zur Trennung eines Gemisches enthaltend mindestens einen Carbonsäurevinylester der allgemeinen Formel R'-C(O)O-CH=CH₂ und mindestens eine Carbonsäure der allgemeinen Formel R'-COOH kann im Anschluß an eine Umvinylierung einer Carbonsäure der allgemeinen Formel R'-COOH mit einem Vinylester der allgemeinen Formel R-C(O)O-CH=CH₂ durchgeführt werden und zur Auftrennung des dabei erhaltenen Reaktionsgemisches eingesetzt werden. Beispielsweise im Anschluß an eine Umvinylierung von Vinylacetat mit Stearinsäure und/oder Palmitinsäure, welche mit einem Ruthenium- oder einem Palladium-Katalysator katalysiert wird. Die Verfahrensbedingungen einer Umvinylierung sind dem Fachmann bekannt, beispielsweise aus der WO 92/09554 A1, der WO 2011/139360 A1, der WO 2011/139361 A1, der WO 2013/117294 A1, der WO 2013/117294 A1, der DE 102013224491 und der DE 102013224496, auf deren diesbezügliche Offenbarung Bezug genommen wird.

Das erfindungsgemäße Verfahren ermöglicht die Trennung beliebiger Gemische aus hochsiedenden Carbonsäurevinylestern und hochsiedenden Carbonsäuren. Durch Überführung der hochsiedenden Carbonsäure in das entsprechende, höher siedende Carbonsäureanhydrid lässt sich der Carbonsäurevinylester abtrennen.

Überraschenderweise wurde gefunden, dass die Überführung der hochsiedenden Carbonsäure in das höher siedende Carbonsäureanhydrid durch Umsetzung mit einem leichtsiedenden Anhydrid in einfacher Weise und mit hoher Selektivität erreicht werden kann.

Überraschenderweise gelingt die Überführung der Carbonsäure in das höher siedende Carbonsäureanhydrid ebenso durch Reaktivdestillation mit einem leichtsiedenden Carbonsäurevinylester in Gegenwart eines Katalysators. Ein verfahrenstechnisch aufwendiger Fest-Flüssig-Trennschritt ist nicht erforderlich. Wird die Auftrennung des hochsiedenden Carbonsäurevinylester/Carbonsäure-Gemisches in Verbindung mit einer Umvinylierungsreaktion durchgeführt, so lässt sich das erhaltene Anhydrid R'-C(O)-O-C(O)-R' der Carbonsäure R'-COOH direkt wieder in einer Umvinylierungsreaktion einsetzen, ohne dass zuvor ein weiterer chemischer Prozesschritt erforderlich ist.

Durch das erfindungsgemäße Verfahren werden hochsiedende Carbonsäurevinylester sowie beliebige Mischungen derselben großtechnisch einfach zugänglich.

### Beispiele:

Die nachfolgenden Beispiele dienen der näheren Erläuterung der Erfindung.

Die angegebenen Zusammensetzungen der Reaktionsgemische wurden mittels quantitativer NMR-Spektroskopie ermittelt.

### Beispiel 1:

### Trennung eines Stearinsäurevinylester/Stearinsäure-Gemisches durch Zugabe von Essigsäureanhydrid

Eine Mischung aus 35 g (113 mmol) Stearinsäurevinylester (ABCR #AB123736) und 15 g (53 mmol) Stearinsäure (Merck #800673) wurde mit 8,1 g (79 mmol) Essigsäureanhydrid (Aldrich #110043) versetzt, 1 Stunde bei 80°C gerührt und anschließend überschüssiges Essigsäureanhydrid und gebildete Essigsäure am Rotationsverdampfer entfernt. Das danach erhaltene Gemisch bestand aus 96,2 Gew.-% Stearinsäurevinylester, 3,2 Gew.-% Stearinsäureanhydrid und 0,6 Gew.-% Stearinsäure. Nach einer Vakuumdestillation bei 100°C - 120°C (0,02 mbar abs.) erhielt man den Stearinsäurevinylester in 99 % Reinheit.

Das Beispiel zeigt, dass durch die Zugabe von Essigsäureanhydrid die Stearinsäure in Stearinsäureanhydrid überführt wird und durch eine nachfolgende Vakuumdestillation Stearinsäurevinylester in hoher Reinheit erhalten werden kann.

### Beispiel 2:

### Trennung eines Stearinsäurevinylester/Stearinsäure-Gemisches aus einer Umvinylierungsreaktion durch Zugabe von Essigsäureanhydrid (in Abwesenheit von Katalysator)

In einem 100 ml Berghoff-Autoklav wurden 20,0 g (70 mmol) Stearinsäure (Merck # 800673), 48,4 g (562 mmol) Vinylacetat (Wacker Chemie AG), 0,068 g (0,3 mmol) Phenothiazin und 0,086 g (0,3 mmol) [Ru₃O(OAc)₆(H₂O)₃]OAc (in Form einer essigsauren Lösung mit 4,5 Gew.-% Ru der Fa. Umicore) bei maximal 10,0 bar abs. 4 Stunden lang auf 140°C erhitzt. Nach dem Abkühlen wurden Essigsäure und Vinylacetat am Rotationsdampfer entfernt. Anschließend wurden Stearinsäurevinylester und Stearinsäure bei 154°C - 173°C (1 mbar abs.) abdestilliert.

Die erhaltenen 18,5 g eines Gemisches aus 94 Gew.-% (17,4 g, 56 mmol) Stearinsäurevinylester und 6 Gew.-% (1,1 g, 4 mmol) Stearinsäure wurde mit 0,59 g (6 mmol) Essigsäureanhydrid (Aldrich # 110043) versetzt, 1 Stunde bei 80°C gerührt und überschüssiges Essigsäureanhydrid sowie die entstandene Essigsäure am Rotationsverdampfer entfernt. Nach erneuter Vakuumdestillation bei 173°C (1 mbar abs.) erhielt man den Stearinsäurevinylester in 99,8% Reinheit.

### Beispiel 3:

### Trennung eines Stearinsäurevinylester/Stearinsäure-Gemisches aus einer Umvinylierungsreaktion durch Zugabe von Essigsäureanhydrid (in Anwesenheit von Katalysator)

In einem 100 ml Berghoff-Autoklav wurden 20,0 g (70 mmol) Stearinsäure (Merck #800673), 48,4 g (562 mmol) Vinylacetat (Wacker Chemie AG), 0,068 g (0,3 mmol) Phenothiazin und 0,086 g (0,3 mmol) [Ru₃O(OAC)₆(H₂O)₃]OAc (in Form einer essigsauren Lösung mit 4,5 Gew.-% Ru der Fa. Umicore) bei maximal 10,0 bar abs. 4 Stunden lang auf 140°C erhitzt. Nach dem Abkühlen wurden Essigsäure und Vinylacetat am Rotationsdampfer entfernt.

Dem Reaktionsgemisch, das 19,2 g (62 mmol) Stearinsäurevinylester und 1,56 g (5,5 mmol) Stearinsäure enthielt, wurden anschließend 1,12 g (11 mmol) Essigsäureanhydrid (Aldrich # 110043) zugesetzt, 1 Stunde bei 80°C gerührt und überschüssiges Essigsäureanhydrid sowie die entstandene Essigsäure am Rotationsverdampfer entfernt. Nach Vakuumdestillation bei 105°C-130°C (0,07 mbar abs.) erhielt man den Stearinsäurevinylester in 99% Reinheit.

### Beispiel 4:

### Trennung eines Palmitinsäurevinylester/Palmitinsäure-Gemisches aus einer Umvinylierungsreaktion durch Reaktivdestillation mit Vinylacetat

In einen 2 l Glasreaktor mit Tauchrohr wurden 220 g/h eines Gemisches aus Palmitinsäure (Carl Roth #5907.2) und Katalysator (aktive Ru-Katalysatorlösung auf Basis von RuCl₃, wie in DE 102014206915 offengelegt, 1000 ppm Ru bezogen auf Palmitinsäure) und 27,7 l/min gasförmiges Vinylacetat eingeleitet. Vinylacetat und entstehende Essigsäure wurden gasförmig abgeführt und außerhalb des Reaktors kondensiert. Die Reaktion wurde bei einem konstanten Füllvolumen des Reaktors von 1750 ml, einer mittleren Verweilzeit von 6,7 h und bei einer Reaktorinnentemperatur von 140°C durchgeführt. Man erhielt ein Gemisch bestehend aus 76 Gew.-% Palmitinsäurevinylester, 20 Gew.-% Palmitinsäureanhydrid und 4 Gew.-% Palmitinsäure. Die Abtrennung des Palmitinsäurevinylesters erfolgte durch eine Vakuumdestillation bei 150°C (1 mbar abs.).

### Beispiel 5:

### Trennung eines Stearinsäurevinylester/Stearinsäure-Gemisches aus einer Umvinylierungsreaktion durch Reaktivdestillation mit Vinylacetat

In einen 2 1 Glasreaktor mit Tauchrohr wurden 225 g/h eines Gemisches aus Stearinsäure (Carl Roth #9459.2) und Katalysator (aktive Ru-Katalysatorlösung auf Basis von RuCl₃, wie in DE 102014206915 offengelegt, 1000 ppm Ru bezogen auf Stearinsäure) und 25,5 l/min gasförmiges Vinylacetat eingeleitet. Vinylacetat und entstehende Essigsäure wurden gasförmig abgeführt und außerhalb des Reaktors kondensiert. Die Reaktion wurde bei einem konstanten Füllvolumen des Reaktors von 1750 ml, einer mittleren Verweilzeit von 6,5 h und bei einer Reaktorinnentemperatur von 140°C durchgeführt. Man erhielt ein Gemisch aus 79 Gew.-% Stearinsäurevinylester, 18 Gew.-% Stearinsäureanhydrid und 3 Gew.-% Stearinsäure. Die Abtrennung des Stearinsäurevinylesters erfolgte durch eine Vakuumdestillation bei 175°C (1 mbar abs.).

## Patentansprüche

1. Verfahren zur Trennung eines Gemisches enthaltend mindestens einen Carbonsäurevinylester der allgemeinen Formel R'-C(O)O-CH=CH₂ und mindestens eine Carbonsäure der allgemeinen Formel R'-COOH, wobei R' jeweils ein aliphatischer Rest mit 12 bis 22 C-Atomen, oder ein cycloaliphatischer Rest mit 12 bis 22 C-Atomen oder ein aromatischer Rest mit 12 bis 22 C-Atomen sein kann und R' gleich oder verschieden sein können, **dadurch gekennzeichnet, dass** die Carbonsäure in deren Anhydrid R'-C(O)-O-C(O)-R' überführt wird und der Carbonsäurevinylester anschließend abgetrennt wird.

2. Verfahren nach Anspruch 1, **dadurch gekennzeichnet, dass** die Überführung der Carbonsäure R'-COOH in das entsprechende Anhydrid R'-C(O)-O-C(O)-R' durch Reaktion mit einem Anhydrid der allgemeinen Formel R*-C(O)-O-C(O)-R* erfolgt, wobei R* gleich oder verschieden ist und einen aliphatischen Rest mit 1 bis 10 C-Atomen, oder einen cycloaliphatischen Rest mit bis zu 10 C-Atomen oder einen aromatischen Rest mit bis zu 10 C-Atomen darstellt.

3. Verfahren nach Anspruch 2, **dadurch gekennzeichnet, dass** 0,1 bis 5 molare Äquivalente des Anhydrids R*-C(O)-O-C(O)-R*, bezogen auf die Carbonsäure R'-COOH, eingesetzt werden und die Reaktion bei Temperaturen von 0°C bis 150°C, einem Druck von 0,001 bis 10 bar abs. und mit einer Reaktionszeit von bis zu 5 Stunden erfolgt.

4. Verfahren nach Anspruch 2 oder 3, **dadurch gekennzeichnet, dass** als Anhydrid der allgemeinen Formel R*-C(O)-O-C(O)-R* ein Anhydrid aus der Gruppe der Anhydride von Essigsäure, Propionsäure, n-Buttersäure, iso-Buttersäure, n-Valeriansäure, 2-Methylbuttersäure, 3-Methylbuttersäure, Pivalinsäure, Capronsäure, Cyclohexancarbonsäure, n-Heptansäure, 2-Methylhexansäure, 2-Ethylhexansäure, n-Octansäure, n-Nonansäure, Isononansäure, Neononansäure, n-Decansäure, Neodecansäure eingesetzt wird.

5. Verfahren nach Anspruch 1, **dadurch gekennzeichnet, dass** die Überführung der Carbonsäure R'-COOH in das entsprechende Anhydrid R'-C(O)-O-C(O)-R' durch Übergangsmetall-katalysierte Reaktivdestillation mit einem Carbonsäurevinylester der allgemeinen Formel R-C(O)O-CH=CH₂ erfolgt, wobei R ein aliphatischen Rest mit 1 bis 10 C-Atomen oder ein cycloaliphatischer Rest mit bis zu 10 C-Atomen oder ein aromatischer Rest mit bis zu 10 C-Atomen sein kann.

6. Verfahren nach Anspruch 5, **dadurch gekennzeichnet, dass** die Reaktivdestillation in Gegenwart von Ru-Verbindungen oder Pd-Verbindungen als Katalysator, bei einem molaren Verhältnis von Carbonsäurevinylester R-C(O)O-CH=CH₂ zu Carbonsäure R'-COOH von 1 : 1 bis 40 : 1, und bei einer Temperatur von 100°C bis 180°C durchgeführt wird.

7. Verfahren nach Anspruch 5 oder 6, **dadurch gekennzeichnet, dass** als Carbonsäurevinylester R-C(O)O-CH=CH₂ ein Carbonsäurevinylester mit einem Alkylrest mit 1 bis 6 C-Atomen als Rest R, vorzugsweise Vinylacetat, eingesetzt wird.

8. Verfahren nach Anspruch 1 bis 7, **dadurch gekennzeichnet, dass** die Abtrennung des Carbonsäurevinylesters mittels Destillation erfolgt.

9. Verfahren nach Anspruch 1 bis 8, **dadurch gekennzeichnet, dass** das Verfahren im Anschluss an eine Umvinylierung einer Carbonsäure der allgemeinen Formel R'-COOH mit einem Carbonsäurevinylester der allgemeinen Formel R-C(O)O-CH=CH₂ durchgeführt wird.

10. Verfahren nach Anspruch 9, **dadurch gekennzeichnet, dass** das Verfahren im Anschluss an eine Umvinylierung von Vinylacetat mit Stearinsäure und/oder Palmitinsäure, welche mit einem Ruthenium- oder einem Palladium-Katalysator katalysiert wird, durchgeführt wird.

## Claims

1. Process for separating a mixture comprising at least one vinyl carboxylate ester of general formula R'-C(O)O-CH=CH₂ and at least one carboxylic acid of general formula R'-COOH, wherein in each case R' may be an aliphatic radical having 12 to 22 carbon atoms or a cycloaliphatic radical having 12 to 22 carbon atoms or an aromatic radical having 12 to 22 carbon atoms and R' may be identical or different, **characterized in that** the carboxylic acid is converted into its anhydride R'-C(O)-O-C(O)-R' and the vinyl carboxylate ester is subsequently removed.

2. Process according to Claim 1, **characterized in that** the conversion of the carboxylic acid R'-COOH into the corresponding anhydride R'-C(O)-O-C(O)-R' is effected by reaction with an anhydride of general formula R*-C(O)-O-C(O)-R*, wherein R* is identical or different and represents an aliphatic radical having 1 to 10 carbon atoms or a cycloaliphatic radical having up to 10 carbon atoms or an aromatic radical having up to 10 carbon atoms.

3. Process according to Claim 2, **characterized in that** 0.1 to 5 molar equivalents of the anhydride R*-C(O)-O-C(O)-R* relative to the carboxylic acid R'-COOH are employed and the reaction is effected at temperatures of 0°C to 150°C, at a pressure of 0.001 to 10 bar abs. and with a reaction time of up to 5 hours.

4. Process according to Claim 2 or 3, **characterized in that** the anhydride of general formula R*-C(O)-O-C(O)-R* employed is an anhydride from the group of anhydrides of acetic acid, propionic acid, n-butyric acid, isobutyric acid, n-valeric acid, 2-methylbutyric acid, 3-methylbutyric acid, pivalic acid, caproic acid, cyclohexanecarboxylic acid, n-heptanoic acid, 2-methylhexanoic acid, 2-ethylhexanoic acid, n-octanoic acid, n-nonanoic acid, isononanoic acid, neononanoic acid, n-decanoic acid, neodecanoic acid.

5. Process according to Claim 1, **characterized in that** the conversion of the carboxylic acid R'-COOH into the corresponding anhydride R'-C(O)-O-C(O)-R' is effected by transition-metal-catalyzed reactive distillation with a vinyl carboxylate ester of general formula R-C(O)O-CH=CH₂, wherein R may be an aliphatic radical having 1 to 10 carbon atoms or a cycloaliphatic radical having up to 10 carbon atoms or an aromatic radical having up to 10 carbon atoms.

6. Process according to Claim 5, **characterized in that** the reactive distillation is performed in the presence of Ru compounds or Pd compounds as catalyst, with a molar ratio of vinyl carboxylate ester R-C(O)O-CH=CH₂ to carboxylic acid R'-COOH of 1 : 1 to 40 : 1 and at a temperature of 100°C to 180°C.

7. Process according to Claim 5 or 6, **characterized in that** the vinyl carboxylate ester R-C(O)O-CH=CH₂ employed is a vinyl carboxylate ester having an alkyl radical having 1 to 6 carbon atoms as radical R, preferably vinyl acetate.

8. Process according to Claim 1 to 7, **characterized in that** removal of the vinyl carboxylate ester is effected by means of distillation.

9. Process according to Claim 1 to 8, **characterized in that** the process is performed following a transvinylation of a carboxylic acid of general formula R'-COOH with a vinyl carboxylate ester of general formula R-C(O)O-CH=CH₂.

10. Process according to Claim 9, **characterized in that** the process is performed following a transvinylation of vinyl acetate with stearic acid and/or palmitic acid which is catalyzed with a ruthenium or palladium catalyst.

## Revendications

1. Procédé pour la séparation d'un mélange contenant au moins un ester vinylique d'acide carboxylique de formule générale R'-C(O)O-CH=CH₂ et au moins un acide carboxylique de formule générale R'-COOH, dans lesquelles R' peut être, à chaque fois, un radical aliphatique comprenant 12 à 22 atomes de carbone ou un radical cycloaliphatique comprenant 12 à 22 atomes de carbone ou un radical aromatique comprenant 12 à 22 atomes de carbone et les radicaux R' peuvent être identiques ou différents, **caractérisé en ce que** l'acide carboxylique est transformé en son anhydride R'-C(O)-O-C(O)-R' et l'ester vinylique d'acide carboxylique est ensuite séparé.

2. Procédé selon la revendication 1, **caractérisé en ce que** la transformation de l'acide carboxylique R'-COOH en anhydride correspondant R'-C(O)-O-C(O)-R' a lieu par réaction avec un anhydride de formule générale R*-C(O)-O-C(O)-R*, dans laquelle les radicaux R* sont identiques ou différents et représentent un radical aliphatique comprenant 1 à 10 atomes de carbone ou un radical cycloaliphatique comprenant jusqu'à 10 atomes de carbone ou un radical aromatique comprenant jusqu'à 10 atomes de carbone.

3. Procédé selon la revendication 2, **caractérisé en ce qu'**on utilise 0,1 à 5 équivalents molaires de l'anhydride R*-C(O)-O-C(O)-R*, par rapport à l'acide carboxylique R'-COOH, et la réaction a lieu à des températures de 0°C à 150°C, à une pression de 0,001 à 10 bars abs. et pendant un temps de réaction de jusqu'à 5 heures.

4. Procédé selon la revendication 2 ou 3, **caractérisé en ce qu'**on utilise comme anhydride de formule générale R*-C(O)-O-C(O)-R* un anhydride du groupe formé par les anhydrides de l'acide acétique, de l'acide propionique, de l'acide n-butyrique, de l'acide iso-butyrique, de l'acide n-valérianique, de l'acide 2-méthylbutyrique, de l'acide 3-méthylbutyrique, de l'acide pivalique, de l'acide caproïque, de l'acide cyclohexanecarboxylique, de l'acide n-heptanoïque, de l'acide 2-méthylhexanoïque, de l'acide 2-éthylhexanoïque, de l'acide n-octanoïque, de l'acide n-nonanoïque, de l'acide isononanoïque, de l'acide néononanoïque, de l'acide n-décanoïque, de l'acide néodécanoïque.

5. Procédé selon la revendication 1, **caractérisé en ce que** la transformation de l'acide carboxylique R'-COOH en anhydride correspondant R'-C(O)-O-C(O)-R' a lieu par distillation réactive catalysée par un métal de transition avec un ester vinylique d'acide carboxylique de formule générale R-C(O)O-CH=CH₂, dans laquelle le radical R peut représenter un radical aliphatique comprenant 1 à 10 atomes de carbone ou un radical cycloaliphatique comprenant jusqu'à 10 atomes de carbone ou un radical aromatique comprenant jusqu'à 10 atomes de carbone.

6. Procédé selon la revendication 5, **caractérisé en ce que** la distillation réactive est réalisée en présence de composés à base de Ru ou de Pd comme catalyseur, à un rapport molaire d'ester vinylique d'acide carboxylique R-C(O)O-CH=CH₂ à acide carboxylique R'-COOH de 1:1 à 40:1 et à une température de 100°C à 180°C.

7. Procédé selon la revendication 5 ou 6, **caractérisé en ce qu'**on utilise, comme ester vinylique d'acide carboxylique R-C(O)O-CH=CH₂ un ester vinylique d'acide carboxylique présentant un radical alkyle comprenant 1 à 6 atomes de carbone comme radical R, de préférence l'acétate de vinyle.

8. Procédé selon la revendication 1 à 7, **caractérisé en ce que** la séparation de l'ester vinylique d'acide carboxylique a lieu par distillation.

9. Procédé selon la revendication 1 à 8, **caractérisé en ce que** le procédé est réalisé après une transvinylation d'un acide carboxylique de formule générale R'-COOH avec un ester vinylique d'acide carboxylique de formule générale R-C(O)O-CH=CH₂.

10. Procédé selon la revendication 9, **caractérisé en ce que** le procédé est réalisé après une transvinylation d'acétate de vinyle avec de l'acide stéarique et/ou de l'acide palmitique, qui est catalysée par un catalyseur à base de ruthénium ou de palladium.
